# EUROPÄISCHE PATENTANMELDUNG

(11) **EP 0 687 665 A1**
(43) Veröffentlichungstag der Anmeldung: **20.12.1995**
(21) Anmeldenummer: 95105567.2
(22) Anmeldetag: 12.04.1995
(51) Int. Cl.: C07C 67/307, C07C 69/63

(54) **Verfahren zur Herstellung von 2,2-Dichlor-malonsäure-diestern**

(30) Priorität: 10.06.1994 DE 4420263
(71) Anmelder: HÜLS AKTIENGESELLSCHAFT, D-45764 Marl (DE)
(72) Erfinder: Theis, Christoph, Dr., D-53859 Niederkassel (DE)

(57) **Zusammenfassung**

Es wird ein Verfahren zur Herstellung von 2,2-Dichlor-malonsäure-diestern durch Umsetzung von Malonsäure-diestern mit wäßrigen Alkali-Hypochlorit-Lösungen oder Erdalkali-Hypochlorit-Suspensionen im pH-Bereich ≧ 8 bei niedrigen Reaktionstemperaturen beschrieben. Mit diesem Verfahren werden hohe Ausbeuten und sehr reine Produkte erzielt.

## Beschreibung

Die vorliegende Erfindung betrifft ein Verfahren zur Herstellung von 2,2-Dichlor-malonsäure-diestern durch Umsetzung von Malonsäure-diestern mit wäßrigen Alkali-Hypochlorit-Lösungen oder Erdalkali-Hypochlorit-Suspensionen im pH-Bereich ≧ 8 bei niedrigen Reaktionstemperaturen.

Dichlor-malonsäure-diethylester werden als Aktivatoren bei der EPM/EPDM-Copolymerisation verwendet (DE-OS 23 44 267 und CA-PS 1 014 299) oder als Flammschutzmittel in Polycarbonaten (DE-OS 24 60 946). Auch als fotographische Entwickler (DE-OS 21 46 430) oder als Zusätze zu Verchromungsbädern (Trans. Int. Met. Finish 1985, 34) finden sie Verwendung.

In der Literatur sind Beispiele für die Synthese von 2,2-Dichlor-malonsäure-diestern, ausgehend vom entsprechenden Malonsäure-diester, beschrieben:
Forster et al. [J. Chem. Soc. 97 (1910), 130] sowie Amriev et al. [Zh. Prikl. Khim. (Leningrad) 1985, 2504] berichten über die Chlorierung von Malonsäure-diethylester mit elementarem Chlor bei erhöhter Temperatur zu einem Gemisch von Monochlor- und überwiegend Dichlor-malonester. Conrad et al. [Chem. Ber. 35 (1902), 1815] führten die Chlorierung von Malonsäure mittels Sulfurylchlorid nebst nachfolgender Veresterung der gebildeten Dichlor-malonsäure durch. Über eine analoge Umsetzung, allerdings mit Thionylchlorid in Essigsäure als Solvens, wird in der CH-PS 599 092 berichtet.

Andere Autoren, wie Just et al. [Tetrahedron Lett. 1979, 3643], führen die Chlorierung von Malonsäure-diethylester mit Trifluormethansulfonsäurechlorid, Hori et al. [Chem. Abstr. 93, 95 231 h (1988)] mit Tetrachlorkohlenstoff in Gegenwart organischer tertiärer N-Basen, Terpighorev et al. [Zh Org. Khim. 1980, 2545] von Malondiester-Mono-Na-Salzen mit Tetrachlorkohlenstoff, Yonemura et al. [Bull. Chem. soc. Jap. 1987, 809] mit dem Redox-System Mn(III)-Acetat/Chlorid durch.

Auch über die Chlorierungen von Monochlor-malonsäure-diester wird berichtet:
So wurde Monochlor-malonsäure-diester von Conrad et al. [Chem. Ber. 24 (1981), 2993] mit elementarem Chlor, von Macbeth et al. [J. Chem. Soc. 121 (1922), 1120 und 2177] mit Sulfurylchlorid, von Shevchenko et al. [Zhur. Obshch. Khim. 32 (1962), 2994] mit Phosphorpentachlorid zum Zielpunkt chloriert.

Allen in der Literatur beschriebenen Verfahren ist gemein, daß die Ausbeuten der gewünschten Zielprodukte meist nur unbefriedigend sind, wobei die Dichlor-Derivate mit unterschiedlichen Mengen Monochlor-Verbindungen verunreinigt sind und daß zur Herstellung der gewünschten Zielprodukte Chlorierungsmittel eingesetzt werden, die aufgrund ihrer Natur oder ihrer bei der gewünschten Umsetzung entstehenden Reaktionsprodukte aus verfahrenstechnischer, wirtschaftlicher und ökologischer Sicht für eine industrielle Fertigung der Zielprodukte wenig geeignet sind.

Es bestand daher die Aufgabe, ein verfahrenstechnisch einfaches, wirtschaftliches und ergiebiges Verfahren zur Herstellung von 2,2-Dichlormalonsäure-diestern aus den zugrundeliegenden und bequem zugänglichen Malonsäure-diestern zu finden.

Überraschenderweise wurde diese Aufgabe durch das erfindungsgemäße Verfahren gelöst, bei dem Malonsäure-diester der allgemeinen Formel I 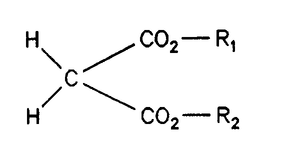
worin R₁ und R₂ die Bedeutung geradkettiges oder verzweigtes C₁- bis C₆-Alkyl, Cycloalkyl und Aralkyl besitzen und die Reste R₁ und R₂ gleich oder verschieden sein können, mit wäßrigen Alkali-Hypochlorit-Lösungen oder Erdalkali-Hypochlorit-Suspensionen bei pH-Werten ≧ 8 bei niedrigen Temperaturen zu den Zielprodukten der allgemeinen Formel II 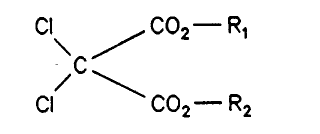
worin R₁ und R₂ die vorgenannte Bedeutung haben, umgesetzt werden.

Die aufgefundene Umsetzung kann beispielsweise bei Verwendung von Natrium-Hypochlorit-Lösung durch folgende formale Reaktionsgleichung beschrieben werden: 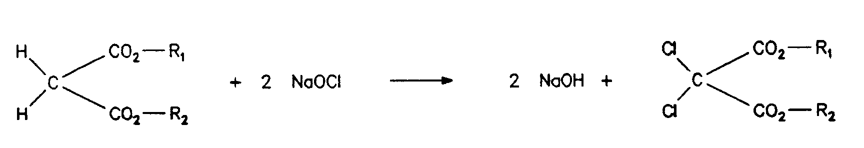
Überraschend war auch, daß das Abpuffern der bei der Umsetzung freiwerdenden Natronlauge durch verdünnte Mineralsäuren, wie Salz-, Schwefel- oder Phosphorsäure, aber auch durch geeignete organische Säuren, wie Ameisensäure, oder substituierte Essig- und Propionsäuren, wie beispielsweise Monochlor-, Dichlor- oder Trichloressigsäure, oder aromatische Carbonsäuren, die mit Wasser verdünnt sein können und die gegebenenfalls einen oder mehrere inerte Substituenten tragen, und Konstanthalten des pH-Werts der Reaktionsmischung im pH-Bereich 8 bis 14, vorzugsweise im pH-Bereich 8,5 bis 14, und besonders bevorzugt im Bereich 9 bis 14, zu extrem hohen Dichlor-malonester-Ausbeuten verbunden mit extrem hohen Produkt-Reinheiten führt.

Der pH-Bereich, in dem die gewünschte Umsetzung durchgeführt wird, liegt in einem breiten Bereich. So sollte zum einen der Stabilität der eingesetzten Hypochlorite durch einen pH-Wert ≧ 8 Rechnung getragen, und zum anderen sollte der pH-Wert zur Erzielung optimaler Ausbeuten, d. h., um Verluste an Edukt und Zielprodukt infolge Verseifungs-Reaktionen zu vermeiden, in der Alkalität des Reaktionsgemisches eingeschränkt werden;
zweckmäßigerweise wird deshalb die Obergrenze des Reaktions-pH-Werts auf ≦ 14 begrenzt. Vorzugsweise wird im pH-Bereich 8,5 bis 14 gearbeitet.

Desweiteren wurde gefunden, daß niedrige Reaktionstemperaturen die erzielbare Ausbeute und Produktreinheit begünstigen. Es wird deshalb zweckmäßigerweise im Temperaturbereich 0 bis 50 °C, vorzugsweise im Temperaturbereich 2 bis 25 °C gearbeitet. Dieser Temperaturbereich gewährleistet eine rasche und schonende Umsetzung der eingesetzten Edukte; die Anwendung tieferer Temperaturen ist aber auch möglich.

Die beschriebene Umsetzung kann beispielsweise so erfolgen, daß das Hypochlorit in Form einer wäßrigen Lösung oder Suspension vorgelegt und das Edukt unter Konstanthaltung des pH-Bereichs, wie vorstehend beschrieben, durch parallel erfolgende Dosierung einer der vorstehend beschriebenen Säuren zudosiert wird, wobei die Säuredosierung auch in der Nachreaktionsphase zur Aufrechterhaltung des gewählten pH-Bereichs fortgesetzt werden kann.

Es kann aber auch eine inverse Arbeitsweise, d. h. Vorlegen des Edukts und Dosierung der Hypochlorit-Lösung nebst Säuredosierung, gewählt werden.

Bei der auszuführenden Malonester-Chlorierung mittels Hypochloriten kann zweckmäßigerweise, bezogen auf die eingesetzten Malonsäureester, ein geringfügiger Überschuß an Hypochlorit eingesetzt werden; der anzuwendende Überschuß ist nicht kritisch. Im allgemeinen werden 2 bis 20 %, vorzugsweise 5 bis 10 %, Hypohalogenit-Überschuß angewandt. Die Malonester-Hypochlorit-Verhältnisse liegen von 1 : 1 bis 1 : 2, vorzugsweise von 1 : 1,05 bis 1 : 1,2. Nach Reaktionsende wird dieser noch vorhandene Hypochlorit-Anteil in bekannter Weise, etwa durch Zugabe gegebenenfalls wäßrigen Natriumsulfits, zerstört.

Im allgemeinen resultiert bei diesen Umsetzungen ein zweiphasiges Reaktionsgemisch, aus dem die organische Phase leicht abgetrennt werden kann. Es ist hierbei zweckmäßig, die resultierende wäßrige Phase unter Verwendung eines geeigneten Extraktionsmittels von noch gelöstem Zielprodukt zu befreien.

Als geeignete Extraktionsmittel gelangen vorzugsweise mit Wasser nicht mischbare Lösemittel, wie Halogenkohlenwasserstoffe, aliphatische, cycloaliphatische und aromatische Kohlenwasserstoffe, Ester und Ether, zum Einsatz, die sich nach Extraktion leicht vom Zielprodukt, etwa durch Normal- oder fraktionierte Vakuum-Destillation, abtrennen lassen.

Die nachfolgenden Beispiele belegen die Anwendungsbreite des erarbeiteten Verfahrens.

### Beispiel 1

217,8 g wäßrige Natrium-Hypochlorit-Lösung (Gehalt: 8,55 % NaOCl entsprechend 250 mMol NaOCl) werden auf 5 °C abgekühlt, der pH-Wert der Lösung wird durch Zugabe wäßrige 20%iger Salzsäure auf ca. 9,1 eingestellt.

Zu dieser gekühlten Vorlage werden im Verlauf von ca. 30 Minuten 36,0 g Malonsäure-diethylester (225 mMol) zugetropft, wobei der pH-Wert der Lösung durch gleichzeitiges Dosieren einer wäßrigen 20%igen Salzsäure im Bereich 9,0 bis 9,2 und die Innentemperatur durch Kühlung bei ca. 5 °C gehalten wird.

Nach beendeter Malonester-Dosierung läßt man unter Konstanthaltung des pH-Bereichs 10 Minuten nachreagieren. Dann wird noch vorhandenes, überschüssiges Natrium-Hypochlorit durch Zugabe von Natriumsulfit zerstört, aus dem 2-Phasen-Gemisch wird die organische Phase abgetrennt, die wäßrige Phase erschöpfend mit tert.-Butylmethyl-ether extrahiert und organische Phase und Extrakte vereinigt.

Nach Eindampfen dieser Lösung wird der resultierende Rückstand einer Vakuum-Destillation unterworfen. Man erhält 50,8 g 2,2-Dichlor-malonsäure-diethylester vom Siedepunkt 95 bis 96 °C (5 hPa), das entspricht einer Ausbeute von 98,6 % d. Th.; die gaschromatografisch ermittelte Reinheit des Produkts beträgt ≧ 99 5 %, der Gehalt an Monochlor-Verbindung ≦ 0,2 %.

### Beispiel 2

Analog Beispiel 1, aber unter Einsatz von 48,6 g Malonsäure-diisobutylester (225 mMol). Der Anfangs- und Reaktions-pH-Bereich beträgt 12,0 bis 12,2.

Nach analoger Aufarbeitung werden 58,0 g 2,2-Dichlor-malonsäure-diisobutylester vom Siedepunkt 114 bis 117 °C (5 hPa) erhalten (Reinheit des Zielprodukts: > 99,3 %), das entspricht einer Ausbeute von 90,4 % d. Th.

### Beispiel 3

Analog Beispiel 1, aber unter Einsatz von 29,7 g Malonsäure-dimethylester (225 mMol).

Durch analoge Aufarbeitung werden 39,9 g 2,2-Dichlor-malonsäure-dimethylester vom Siedepunkt 72 bis 73 °C (5 hPa) erhalten (Reinheit: > 99,5 %), das entspricht einer Ausbeute von 88,2 % d. Th.

### Beispiel 4

Analog Beispiel 1, aber unter Einsatz von 42,3 g Malonsäure-diisopropylester (225 mMol). Die Reaktion wird bei pH 13 gestartet und im Bereich 13,0 bis 13,5 durchgeführt, wobei die Nachreaktionszeit ca. 60 Minuten beträgt.

Durch analoge Aufarbeitung werden 52,5 g 2,2-Dichlor-malonsäure-diisopropylester vom Siedepunkt 92 bis 93 °C (5 hPa) erhalten (Reinheit: ≧ 99,5 %), das entspricht einer Ausbeute von 90,8 % d. Th.

### Beispiel 5

Analog Beispiel 4, aber unter Einsatz von 42,3 g Malonsäure-tert.-butylethylester (225 mMol). Die Umsetzung und Nachreaktion wird im Temperaturbereich 20 bis 22 °C durchgeführt.

Nach analoger Aufarbeitung werden 48,8 g 2,2-Dichlor-malonsäure-tert.-butylethylester vom Siedepunkt 95 bis 96 °C (5 hPa) erhalten (Reinheit: > 98 %), das entspricht einer Ausbeute von 84,4 % d. Th.

### Beispiel 6

Analog Beispiel 4, aber unter Einsatz von 39,2 g Malonsäure-tert.-butylmethylester (225 mMol).

Nach analoger Aufarbeitung werden 46,8 g 2,2-Dichlor-malonsäure-tert.-butylmethylester vom Siedepunkt 86 bis 88 °C (5 hPa) erhalten (Reinheit: > 99 %), das entspricht einer Ausbeute von 85,2 % d. Th.

## Patentansprüche

1. Verfahren zur Herstellung von 2,2-Dichlor-malonsäure-diestern der allgemeinen Formel 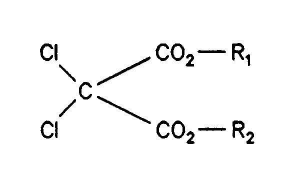 worin R₁ und R₂ geradkettiges oder verzweigtes Alkyl mit 1 bis 6 C-Atomen, Cycloalkyl und Aralkyl bedeuten und die Reste R₁ und R₂ gleich oder verschieden sein können, durch Umsetzung von Malonsäure-diestern der allgemeinen Formel 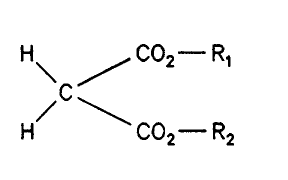 worin R₁ und R₂ geradkettiges oder verzweigtes C₁- bis C₆-Alkyl, Cycloalkyl und Aralkyl bedeuten und die Reste R₁ und R₂ gleich oder verschieden sein können, mit gegebenenfalls überschüssigen wäßrigen Alkali- oder Erdalkali-Hypochlorit-Lösungen im alkalischen Milieu bei pH-Werten von 8 bis 14 und niedrigen Temperaturen von unter 0 bis 50 °C unter gleichzeitiger Konstanthaltung des gewählten pH-Bereichs durch parallel erfolgende Zugabe einer geeigneten Säure.

2. Verfahren nach Anspruch 1,
dadurch gekennzeichnet,
daß die Umsetzungen bei pH-Werten von 8,5 bis 14, vorzugsweise von 9 bis 14, durchgeführt werden.

3. Verfahren nach den Ansprüchen 1 und 2,
dadurch gekennzeichnet,
daß als Säuren zur Konstanthaltung des gewählten pH-Bereichs verdünnte wäßrige Mineralsäuren, wie Salz-, Schwefel- oder Phosphorsäure, oder aliphatische Carbonsäuren, die gegebenenfalls mit Wasser verdünnt und mit einem unter den Reaktionsbedingungen inerten Rest substituiert sein können, wie beispielsweise Ameisen-, Essig- oder Propionsäure oder Monochlor-, Dichlor- oder Trichlor-essigsäure, oder verdünnte aromatische Carbonsäuren, die gegebenenfalls mit einem oder mehreren inerten Resten substituiert sein können, eingesetzt werden.

4. Verfahren nach den Ansprüchen 1 bis 3,
dadurch gekennzeichnet,
daß die Umsetzungen im Temperaturbereich von 2 bis 25 °C durchgeführt werden.

5. Verfahren nach den Ansprüchen 1 bis 4,
dadurch gekennzeichnet,
daß die Umsetzungen mit einem Malonester-Hypochlorit-Verhältnis von 1 : 1 bis 1 : 2, vorzugsweise mit einem Verhältnis von 1 : 1,05 bis 1 : 1,2, durchgeführt werden.

6. Verfahren nach den Ansprüchen 1 bis 5,
dadurch gekennzeichnet,
daß die Hypochlorite als wäßrige Lösungen oder als wäßrige Suspensionen eingesetzt werden.

7. Verfahren nach den Ansprüchen 1 bis 6,
dadurch gekennzeichnet,
daß je nach gewählter Verfahrensweise einer der beiden Reaktionspartner während der Umsetzung zum Reaktionsgemisch dosiert wird.
